(19) Europäisches Patentamt
European Patent Office
Office européen des brevets

(11) Numéro de publication : **0 287 482 B1**

(12) **FASCICULE DE BREVET EUROPEEN**

(45) Date de publication du fascicule du brevet : **10.07.91 Bulletin 91/28**

(51) Int. Cl.$^5$ : **A61L 29/00**, A61L 33/00, A61L 31/00

(21) Numéro de dépôt : **88420114.6**

(22) Date de dépôt : **07.04.88**

(54) **Articles à base de polychlorure de vinyle plastifié pour le contact avec les milieux biologiques.**

Le dossier contient des informations techniques présentées postérieurement au dépôt de la demande et ne figurant pas dans le présent fascicule.

(30) Priorité : **16.04.87 FR 8705646**

(43) Date de publication de la demande : **19.10.88 Bulletin 88/42**

(45) Mention de la délivrance du brevet : **10.07.91 Bulletin 91/28**

(84) Etats contractants désignés : **AT BE CH DE ES FR GB GR IT LI LU NL SE**

(56) Documents cités :
**FR-A- 2 325 672**
**US-A- 4 529 711**
**CHEMICAL ABSTRACTS, vol. 100, no. 22, 28 mai 1984, page 41, résumé no. 175896g, Columbus, Ohio, US; & JP-A-58 167 693 (TORAY SILICONE CO., LTD) 03-10-1983**

(73) Titulaire : **RHONE-POULENC CHIMIE**
**25, quai Paul Doumer**
**F-92408 Courbevoie Cédex (FR)**

(72) Inventeur : **Bartholomay, Eric**
**68, Grande-Rue**
**F-78240 Chambourcy (FR)**
Inventeur : **Gay, Michel**
**11, rue Garibaldi**
**F-69006 Lyon (FR)**
Inventeur : **Jaubert, Jean-Pierre**
**Fontaine Saint-Martin H2**
**F-95350 Saint-Brice-Sous-Foret (FR)**

(74) Mandataire : **Seugnet, Jean Louis et al**
**RHONE-POULENC INTERSERVICES Service Brevets Chimie Centre de Recherches des Carrières B.P. 62**
**F-69192 Saint-Fons Cédex (FR)**

**EP 0 287 482 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne les divers articles (tubes de circulation extracorporelle, cathéters, poches à sang et à soluté) à base de polychlorure de vinyle (PVC) plastifié adapté au contact des milieux biologiques en particulier les liquides biologiques tels que le sang et ses dérivés (plaquettes sanguines, plasma), ainsi que les liquides nutritionnels (solutés, lipides, etc.....), administrés par voie intraveineuse.

Du fait de son faible coût et de sa facilité à être transformé en objets de formes diverses par les méthodes classiques de transformation des matières plastiques (extrusion, extrusion soufflage, extrusion moulage, calendrage, injection), le PVC plastifié est devenu un matériau de base important dans le domaine médical.

A l'heure actuelle c'est bien souvent en PVC plastifié que sont réalisés divers articles, souvent à usage unique, en particulier les poches à sang, les poches à soluté et les poches à administration parentérale, les cathéters et les tubes de circulation extracorporelle.

En dehors de l'application médicale spécifique, il est connu depuis longtemps d'ajouter un additif organopolysiloxane au PVC en vue de l'utiliser comme lubrifiant externe du PVC rigide ou du PVC plastifié ou comme débullant du plastisol (mélange pâteux de plastifiant et de PVC).

Comme littérature illustrant ce type d'additif pour PVC, on peut citer la demande de brevet japonais Kokai 55/73 742, publiée le 3 juin 1980, l'additif silicone étant une huile diméthylpolysiloxane, le brevet américain US-A-4 512 916, l'additif silicone étant une huile $\alpha$, $\omega$-bis(hydroxyalkyl)diorganopolysiloxane, la demande de brevet japonais Kokai 58/180 555, publiée le 22 octobre 1983, l'additif silicone étant une huile diorganopolysiloxane à fonctions organiques diverses (amino, époxy, carboxyle, alcool, etc.....) la demande de brevet japonais KOKAI 58/167 693, l'additif silicone étant une huile diorganopolysiloxane comportant par molécule, deux groupes organofonctionnels différents dont un des deux groupes peut éventuellement être un groupe hydroxyalkyle et le brevet américain US-A-4 231 909, l'additif silicone étant un fluide organopolysiloxane présentant au moins 10 % molaire de motifs 3-chloropropylsiloxy.

Selon le brevet européen EP-A-43 965, l'additif silicone peut être non seulement un fluide mais un solide c'est-à-dire une résine silicone dont les motifs siloxanes se présentent sous une forme ramifiée ou en réseau.

Dans l'application médicale spécifique, on trouve essentiellement dans la littérature des additifs qui sont des copolymères séquencés ou à blocs d'organopolysiloxane et de polymères organiques variés tels que des polyuréthannes, des polyesters, des polylactones, des polyéthers et des polyurées.

Parmi les brevets illustrant ce type d'additifs on peut citer le brevet britannique GB-A-1 547 834, les brevets français FR-A-2 017 527, FR-A-2 491 938 et FR-A-2 497 217 et le brevet américain US-A-4 636 552.

Comme indiqué dans ces brevets des copolymères d'organopolysiloxanes sont connus en soi pour migrer en grande partie à la surface du PVC et pour conférer à cette surface une énergie de surface ou encore une tension superficielle critique qui permettrait d'éviter la coagulation du sang. Cependant, comme indiqué dans ces brevets, les organopolysiloxanes ont tendance à exsuder à l'extérieur du PVC et présentent chimiquement une très mauvaise compatibilité avec le sang. Cette très mauvaise compatibilité est illustrée par exemple dans le cas des fluides organopolysiloxane à fonctionnalité hydroxyalkyle ou carboxyalkyle, par le brevet belge BE-A-891 629. Selon les brevets ci-dessus ce double inconvénient des organopolysiloxanes est limité par l'utilisation de copolymères à blocs ou séquencés qui permettrait d'aboutir à un compromis acceptable. Le brevet FR-A-2 325 672 décrit l'utilisation de compositions de PVC et d'un additif, tel que l'huile silicone, pour la fabrication d'articles médicaux, mais les huiles silicones utilisées en tant qu'additif semblent être libérées (relarguées) au contact des liquides biologiques et les articles médicaux fabriqués à partir de ces compositions sont opaques, ce qui présente un inconvénient dans le cas des cathéters et des poches à sang.

En outre il subsiste un problème capital non résolu par les compositions de PVC connues.

Ce problème concerne la migration des plastifiants du PVC dans les liquides biologiques, illustré par exemple par l'article de la revue TOXICOLOGY, 9 (1978), pages 319-329.

Un but de la présente invention est, précisément, de proposer un article à base d'une composition de PVC adaptée au contact des milieux biologiques, en particulier le sang et ses dérivés tels que le plasma et les plaquettes sanguines, et les liquides nutritionnels administrés par voie intraveineuse.

Un autre but de la présente invention est de proposer un article à base d'une composition de PVC plastifié comportant un additif silicone conférant un aspect de surface et une énergie de surface satisfaisant pour le contact avec les liquides biologiques sans pour autant être libéré (relargué) au contact de ces liquides.

Un autre but de la présente invention est de proposer un article à base d'une composition de PVC plastifié dont le plastifiant n'est pas libéré (relargué) au contact des liquides biologiques.

Un autre but de la présente invention est de proposer un article à base d'une composition de PVC plastifié dont l'additif silicone, en plus de son rôle d'agent conférant une hémocompatibilité satisfaisante, joue également le rôle de lubrifiant interne et externe du PVC, sans pour cela détériorer la soudabilité du PVC, la composition de PVC demeurant thermoscellable et en outre collable et imprimable.

Un autre but de la présente invention est de proposer des articles en matériau souple étant transparents, présentant une excellente perméabilité à l'oxygène et possédant une tenue au froid pouvant aller jusqu'à −100°C, propriété indispensable pour réaliser des poches à sang conservées généralement à −80°C.

Ces buts et d'autres sont atteints par la présente invention qui concerne en effet un article transparent formé d'une composition de PVC et dont au moins une partie de la surface est destinée à être en contact avec un milieu biologique ou un liquide injectable, caractérisé en ce que la composition de PVC comporte :

- 100 parties de résine PVC,
- 10 à 90 (de préférence 20 à 85) parties d'au moins un plastifiant organique, et
- 0,005 à 3 parties, de préférence de 0,05 à 1,5 partie, d'au moins un diorganopolysiloxane de formule moyenne :

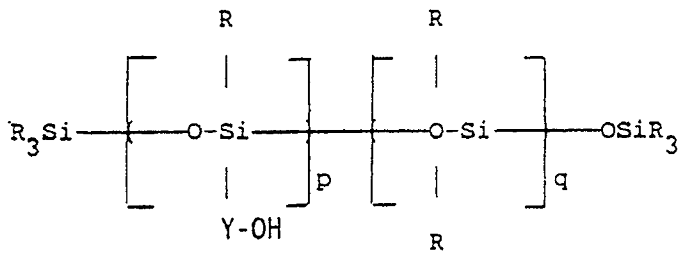

(1)

dans laquelle les groupes R, identiques ou différents, sont choisis parmi les radicaux méthyle et phényle, 60% molaire, de préférence 90%, au moins des radicaux R étant des radicaux méthyle, le groupe Y est un chaînon alkylène divalent hydrocarboné linéaire ou ramifié comportant de 1 à 18 atomes de carbone inclus, de préférence de 1 à 4 atomes inclus, p est un nombre entier compris entre 1 et 50, de préférence entre 2 et 25 et q est un nombre entier compris entre 2 et 400 inclus, de préférence entre 5 et 100 inclus.

Dans ce qui suit ou ce qui précède les pourcentages et parties sont en poids, sauf mentions contraires.

Il est très surprenant que l'additif diorganopolysiloxane de formule (1) confère une bonne hémocompatibilité à la résine PVC étant donné que ce même additif est connu par le brevet belge BE-A-891 629 comme promoteur de coagulation du sang.

Les additifs diorganopolysiloxane sont des produits connus et un certain nombre d'entre eux sont disponibles dans le commerce.

Ces produits sont notamment décrits avec parfois leurs procédés de préparation dans les brevets américains US-A-2 970 150, US-A-4 160 775 et US-A-4 636 552, le brevet belge BE-A-891 629, la demande de brevet japonais précitée Kokai 58/180 555, et la demande de brevet français 85/16 334 déposée le 30 octobre 1985, cités comme référence.

Les additifs silicone de formule (1) dans laquelle R est un méthyle sont les additifs préférés.

Par résine PVC on entend selon l'invention essentiellement, les homopolymérisats. Toutefois la présence de copolymères du chlorure de vinyle n'est pas exclue dans la proportion d'au plus 50 parties de copolymérisat pour 100 parties d'homopolymérisats.

Par milieu biologique on entend selon l'invention les tissus, cellules et liquides biologiques tels que le lait, le sperme, le sang et ses dérivés, en particulier les plaquettes sanguines et le plasma ainsi que les liquides injectables tels que les sérums physiologiques contenant éventuellement des sucres et des médicaments et les liquides nutritionnels que l'on administre au patient par hémodialyse.

Par article adapté pour le contact avec les milieux biologiques on entend selon l'invention notamment les prothèses internes temporaires, les récipients et poches à conservation d'organes et de cellules telles que les plaquettes sanguines, les tubes de circulation extracorporelle, les cathéters, les poches à sang et à soluté, certains de ces articles étant à usage unique.

Les plastifiants utilisés sont les plastifiants usuels du PVC parmi lesquels on peut citer plus particulièrement le diisooctylphtalate (DOP), le diisooctyladipate (DOA), le diisooctylsébaçate (DOS), le diéthylhexylphtalate (DEHP) et le trioctyltrimélitate (TOTM) et leurs divers mélanges possibles.

Les compositions de résine PVC peuvent comporter en outre divers additifs usuels tels que des stabilisants tels que des ß-dicétones, des sels de calcium et de zinc comme le stéarate ou l'octoate de zinc et le stéarate de calcium, et des lubrifiants tels que l'huile de soja époxydée, les cires et l'huile de ricin hydroxylée.

Le mélange de résine PVC et de l'additif silicone peut être effectué de différentes façons. On peut par exemple effectuer le mélange de ces deux produits dans un solvant organique et ensuite éliminer le solvant. Selon une variante on peut mettre en solution dans un solvant organique la résine PVC d'une part et l'additif silicone d'autre part, mélanger les deux solutions puis éliminer le solvant. Le mélange obtenu est alors ajouté au plastifiant et aux additifs.

On peut également faire le mélange directement en utilisant des moyens mécaniques classiques, tels que malaxeur, pétrin, et mélangeurs rapides du type Hopenmeier® et Henschel®, etc...

Dans ce cas, selon une variante préférée, on disperse au préalable l'additif silicone dans le plastifiant puis on mélange mécaniquement le plastifiant à la résine PVC, ainsi qu'éventuellement les autres additifs.

Les articles selon l'invention sont préparés par les procédés classiques d'extrusion, extrusion soufflage, extrusion moulage, d'injection moulage et de calendrage, suivi éventuellement de thermoscellage, collage et impression.

D'autres avantages et caractéristiques de la présente invention apparaîtront à la lecture des exemples suivants, donnés à titre illustratif nullement limitatif.

— EXEMPLE 1 :

Préparation d'une huile diméthylpolysiloxane à motifs γ-hydroxypropyle dans la chaîne :

Dans un ballon tricol de 1 litre, on charge 500 g d'une huile hydrogénométhydiméthylpolysiloxane bloquée à chacune des deux extrémités de la chaîne par un motif triméthylsilyle. Cette huile porte en moyenne 9 motifs hydrogénométhylsiloxane et 7,3 motifs diméthylsiloxane par molécule dans la chaîne. La masse moléculaire en nombre est de 1250.

On porte la charge à 120°C et on introduit de l'alcool allylique et un catalyseur au platine qui est un complexe de platine et de tétravinyltétraméthylcyclotétrasiloxane préparé conformément à l'exemple 3 du brevet américain US-A-3 715 334, à la dose de 8 mg de platine par kg de polysiloxane. L'alcool allylique est ajouté suivant un excès molaire de 50% de groupes OH par rapport aux groupes SiH de l'huile suivant le mode opératoire ci-après :

On charge une ampoule de coulée avec 80% de l'alcool à introduire et renfermant la totalité du catalyseur, 20% de ce mélange est coulé puis on ajoute le restant d'alcool, le mélange réactionnel étant maintenu au reflux. L'addition s'effectue en 4 heures. Les SiH résiduaires sont dosés à la fin de l'addition de l'alcool et la réaction est poursuivie jusqu'à disparition totale des SiH dans la masse réactionnelle (durée 3 heures). L'excès d'alcool est éliminé en maintenant le chauffage pendant 30 minutes sous pression réduite (1,33 Kpa).

L'huile ainsi obtenue est colorée et trouble, après traitement au noire de carbone et filtration sur terre de diatomée Clarcel® on obtient une huile incolore et limpide avec un rendement en hydroxyle pratiquement quantitatif, une masse moléculaire en nombre de 1750 et un pourcentage en poids d'hydroxyle de 8%.

— EXEMPLE COMPARATIF 2 ET EXEMPLES 3 A 5 :

On prépare le mélange suivant :

| | | |
|---|---|---|
| - PVC EKAVYL ® valeur de K 70 (IP = 122-132) | : | 100 parties |
| - diisooctyladipate (DOA) | : | 45 parties |
| - huile de soja époxydée | : | 5 parties |
| - stéarate de calcium | : | 0,25 partie |
| - stéarate de zinc | : | 0,25 partie |
| - huile γ-hydroxypropylée préparée à l'exemple 1 | : | X |

On effectue le mélange des constituants ci-dessus en faisant varier le teneur X de l'huile γ-hydroxypropylée. Pour l'exemple comparatif 2, X = 0 ; exemple 3, X = 0,5 partie ; exemple 4, X = 1,1 partie ; exemple 5, X = 1,3 partie.

On fabrique à partir de ces différents mélanges des cathéters de diamètre intérieur 0,9 mm et de diamètre extérieur 2,0 mm. Le cathéter préparé suivant la composition de l'exemple comparatif 2 est le cathéter témoin.

— EXEMPLE 6 :

Analyse chimique des surfaces internes des cathéters.

La technique utilisée est l'ESCA ("Electron Spectroscopy For Chemical Analysis") dénommée également XPS ("X ray Photoelectron Spectroscopy").

Cette technique connue permet l'analyse élémentaire semi-quantitative de la surface interne des cathéters sur une profondeur inférieure à 100 $10^{-10}$ m. Les cathéters sont ouverts et aplatis par compression pendant 24 heures pour que l'échantillon présente une surface interne plane.

En des points différents de la surface interne de chaque cathéter des exemples 2 à 5, on enregistre un spectre général en haute résolution entre 0 et 1000 eV afin de détecter les éléments et la composition atomique.

Le spectromètre utilisé est le spectromètre SSL à microfaisceau de rayons X (spot de 300 µm). Les mesures sont effectuées sous une pression réduite comprise entre environ $5.10^{-7}$ et $10^{-6}$ Pa.

Les résultats moyens obtenus sont rassemblés dans le tableau 1 ci-après, dans lequel pour le silicium et le chlore on indique le pourcentage atomique (% A) et l'énergie de liaison (E.L.) en eV.

TABLEAU 1

| CATHETER | SILICIUM | | CHLORE | |
|---|---|---|---|---|
| | E.L. | % A | E.L. | % A |
| Témoin ex. 2 | - | - | 200,1 | 7,6 |
| ex. 3 | 101,9 | 14,4 | - | < 1 |
| ex. 4 | 102,2 | 19,3 | - | traces |
| ex. 5 | 102,1 | 17,6 | - | 1,2 |

Du tableau 1 il apparaît que le cathéter témoin de l'exemple comparatif 2 est bien exempt de silicium.

Les cathéters des exemples 3, 4 et 5 ne présentent pratiquement plus d'atome de chlore, ce qui prouve que l'additif silicone recouvre bien la surface interne du cathéter.

— EXEMPLE 7 :

Mise en évidence de l'hémocompatibilité.

L'hémocompatibilité est mise en évidence par le test de DUDLEY tel que décrit par DUDLEY B., Synthesis and Characterization of blood compatible surfaces, Trans. Am. Soc. Artif. Int. Organs, vol. 22, page 538 (1976).

Suivant ce test on implante le cathéter à tester et le cathéter témoin respectivement dans chacune des veines dorsales des pattes avant gauche et droite d'un chien maintenu sous anesthésie. On ligature la veine au niveau de l'implantation du cathéter. On effectue le test de DUDLEY pendant plusieurs minutes, les cathéters sont ensuite fermés avec une pince métallique puis ouverts à différents temps afin de tester leur perméabilité et doser la fibrinopeptide A (FPA) de l'échantillon de sang recueilli.Le débit sanguin est au début du test de l'ordre de 4 ml/mn. Le dosage de FPA est effectué par le kit de mesure immuno-enzymatique du FPA Asserachrom ® FPA commercialisé par la Société STAGO.

Ce kit de mesure est spécifique du FPA humain mais des essais ont montré qu'il était utilisable pour la

mesure du FPA du chien. Il est bien connu que la mesure du FPA est actuellement le paramètre le plus sensible et le plus précoce permettant de révéler l'activation de la coagulation.

Les essais ont été effectués sur les cathéters des exemples 3 et 4, comparés au cathéter témoins de l'exemple comparatif 2 et sur deux chiens différents.

Les résultats obtenus sont rassemblés dans le tableau 2 ci-après dans lequel la teneur en FPA est indiquée en nanogramme par ml de sang, le débit Q du sang est indiqué en ml par minute et le temps d'écoulement T du sang est indiqué en minute.

TABLEAU 2

| | CHIEN n° 1 | | | | CHIEN n° 2 | | | |
|---|---|---|---|---|---|---|---|---|
| CATHETER | EXEMPLE 2 | | EXEMPLE 3 | | EXEMPLE 2 | | EXEMPLE 3 | |
| T | F | Q | F | Q | F | Q | F | Q |
| 0 | 180 | 4 | 194 | 4,4 | 200 | 2,9 | 148 | 4 |
| 3 | 32 | 4 | 14 | 4,1 | | | | |
| 6 | 100 | 3,3 | 78 | 3,0 | 80 | 2,7 | 50 | 3,5 |
| 7 | 144 | 2,4 | | | | | | |
| 8 | - | 0,96 | 144 | 2,7 | | | | |
| 9 | | | | | 300 | 2,5 | | |
| 10 | | | | | 336 | 2,1 | | |
| 11 | | | | | 420 | 1,3 | | |
| 12 | | | | | | | 195 | 3,5 |
| 15 | | | | | | | 400 | 3,3 |
| 16 | | | | | | | 430 | 2,5 |
| 17 | | | | | | | 500 | 1,9 |
| 18 | | | | | | | 780 | 0,8 |

Du tableau 2, il apparaît que l'additif silicone améliore sensiblement l'hémocompatibilité.

— EXEMPLE 8 :

Dans cet exemple en évalue l'extraction, par du plasma de boeuf, de l'additif silicone des cathéters des exemples 3, 4 et 5.

L'extraction est effectuée par immersion de 7,3 g de cathéter à 37°C dans 50 ml de plasma de boeuf pendant 4 heures.

Le dosage montre que tous les cathéters des exemples 3, 4 et 5 présentent une quantité d'additif silicone extraite, inférieure à 0,1% en poids de la quantité totale d'additif silicone introduite dans la composition PVC de départ.

## Revendications

1. Article transparent formé d'une composition de PVC et dont au moins une partie de la surface est destinée à être en contact avec un milieu biologique ou un liquide injectable, caractérisé en ce que la composition de PVC comporte :

- 100 parties de résine PVC,
- 10 à 90 parties d'au moins un plastifiant organique, et
- 0,005 à 3 parties, de préférence de 0,05 à 1,5 partie, d'au moins un diorganopolysiloxane de formule moyenne :

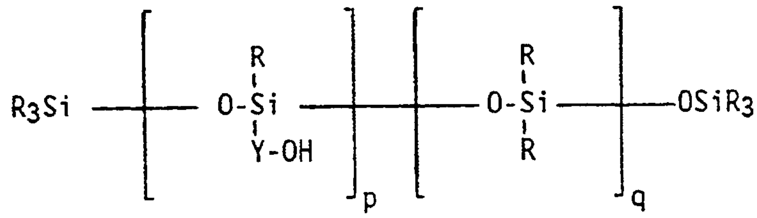

(1)

dans laquelle les groupes R, identiques ou différents, sont choisis parmi les radicaux méthyle et phényle, 60% molaire, de préférence 90%, au moins des radicaux R étant des radicaux méthyle, le groupe Y est un chaînon alkylène divalent hydrocarboné linéaire ou ramifié comportant de 1 à 18 atomes de carbone inclus, de préférence de 1 à 4 atomes inclus, p est un nombre entier compris entre 1 et 50, et q est un nombre entier compris entre 2 et 400 inclus.

2. Article selon la revendication 1, caractérisé en ce que la composition PVC comporte :

- 100 parties de résine PVC,
- 20 à 85 parties au moins d'un plastifiant organique, et
- de 0,05 à 1,5 partie d'au moins un diorganopolysiloxane de formule (1) dans laquelle :
- R est un méthyle,
- Y est une chaîne alkylène ayant de 1 à 4 atomes de carbone,
- p est compris entre 2 et 25 inclus,
- q est compris entre 5 et 100 inclus.

3. Article selon la revendication 1, caractérisé en ce que ledit article est choisi parmi un tube de circulation extracorporelle, un cathéter et une poche à sang ou à soluté.

4. Procédé de préparation d'un article tel que défini à l'une quelconque des revendications 1 à 3, caractérisé en ce qu'on disperse le diorganopolysiloxane de formule (1) dans le plastifiant organique, puis on ajoute et on mélange la résine PVC et éventuellement les autres additifs.

## Claims

1. Transparent article made of a PVC composition and at least a part of the surface of which is intended to come into contact with a biological medium or an injectable liquid, characterised in that the PVC composition comprises :

- 100 parts of PVC resin,
- 10 to 90 parts of at least one organic plasticiser, and
- 0.005 to 3 parts, preferably from 0.05 to 1.5 parts, of at least one diorganopolysiloxane of average formula :

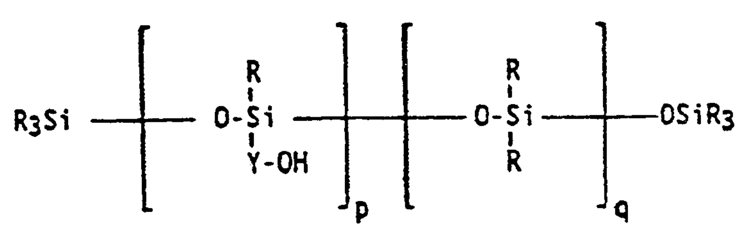

(1)

in which the groups R, which are identical or different, are chosen from methyl and phenyl radicals, at least 60 mol%, preferably 90%, of the radicals R being methyl radicals, the group Y is a linear or branched divalent alkylene hydrocarbon chain unit containing from 1 to 18 carbon atoms inclusive, preferably from 1 to 4 atoms inclusive, p is an integer between 1 and 50 and q is an integer between 2 and 400 inclusive.

2. Article according to claim 1, characterised in that the PVC composition comprises :

– 100 parts of PVC resin

– at least 20 to 85 parts of an organic plasticiser, and

– from 0.05 to 1.5 parts of at least one diorganopolysiloxane of formula (1) in which :

– R is a methyl,

– Y is an alkaline chain containing from 1 to 4 carbon atoms,

– p is between 2 and 25 inclusive,

– q is between 5 and 100 inclusive.

3. Article according to claim 1, characterised in that the said article is chosen from an extracorporeal circulation tube, a catheter and a blood or solution pouch.

4. Process for preparing an article such as defined in any one of claims 1 to 3, characterised in that the diorganopolysiloxane of formula (1) is dispersed in the organic plasticiser, and then the PVC resin and optionally the other additives are added and mixed.

## Patentansprüche

1. Transparentes Erzeugnis, gebildet aus einer PVC-Zusammensetzung, von der wenigstens ein Teil der Oberfläche dazu bestimmt ist, in Kontakt mit einem biologischen Milieu oder einer injizierbaren Flüssigkeit zu sein, dadurch gekennzeichnet, daß die PVC-Zusammensetzung enthält :

100 Teile PVC-Harz,

10 bis 90 Teile mindestens eines organischen Weichmachers und

0,005 bis 3 Teile, vorzugsweise 0,05 bis 1,5 Teile, mindestens eines Diorganopolysiloxans der mittleren Formel

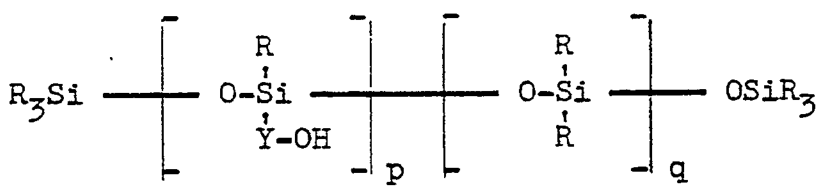

(1)

worin die Gruppen R, die identisch oder verschieden sind, ausgewählt sind unter den Methyl- und Phenylresten, wobei 60 Mol-%, vorzugsweise 90 Mol-%, mindestens der Reste R Methylreste sind, die Gruppe Y ein lineares oder verzweigtes, zweiwertiges Akylen-Kohlenwasserstoffkettenglied mit 1 bis einschließlich 18 Kohlenstoffatomen, vorzugsweise von 1 bis einschließlich 4 Atomen, ist, p eine ganze Zahl zwischen 1 und 50 ist und q eine ganze Zahl zwischen 1 und einschließlich 400 ist.

2. Erzeugnis gemäß Anspruch 1, dadurch gekennzeichnet, daß die PVC-Zusammensetzung enthält :

100 Teile PVC-Harz,

20 bis 85 Teile mindestens eines organischen Weichmachers und

0,05 bis 1,5 Teile mindestens eines Diorganopolysiloxans der Formel (1), worin

R ist ein Methyl,

Y ist eine Alkylenkette mit 1 bis 4 Kohlenstoffatomen,

p ist zwischen 2 und einschließlich 25,

q ist zwischen 5 und einschließlich 100.

3. Erzeugnis gemäß Anspruch 1, dadurch gekennzeichnet, daß dieses Erzeugnis ausgewählt ist unter einem Schlauch bzw. Röhre für Zirkulation außerhalb des Körpers, einem Katheter und einer Tasche für Blut oder Gelöstes.

4. Verfahren zur Herstellung eines Erzeugnisses, wie in einem der Ansprüche 1 bis 3 definiert, dadurch gekennzeichet, daß man das Diorganopolysiloxan der Formel (1) in dem organischen Weichmacher dispergiert und dann das PVC-Harz und gegebenenfalls die anderen Zusätze zugibt und mischt.